(19) Europäisches Patentamt
European Patent Office
Office européen des brevets

(11) **EP 2 606 147 B1**

(12) **EUROPÄISCHE PATENTSCHRIFT**

(45) Veröffentlichungstag und Bekanntmachung des Hinweises auf die Patenterteilung:
**15.10.2014 Patentblatt 2014/42**

(21) Anmeldenummer: **11738632.6**

(22) Anmeldetag: **30.07.2011**

(51) Int Cl.:
*C12Q 1/58* (2006.01)   *G01N 33/543* (2006.01)

(86) Internationale Anmeldenummer:
**PCT/EP2011/003837**

(87) Internationale Veröffentlichungsnummer:
**WO 2012/022428 (23.02.2012 Gazette 2012/08)**

(54) **VERFAHREN ZUR ISOLIERUNG VON HARNSTOFF UNTER ENTFERNUNG VON STÖRENDEM CO2**

METHOD FOR ISOLATING UREA WHILE REMOVING OBJECTIONABLE CO2

PROCÉDÉ D'ISOLEMENT D'URÉE AVEC ÉLIMINATION DE CO2 GÉNANT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **07.12.2010 EP 10193920**
**20.08.2010 EP 10173593**

(43) Veröffentlichungstag der Anmeldung:
**26.06.2013 Patentblatt 2013/26**

(73) Patentinhaber: **Cytonet GmbH & Co. KG**
**69469 Weinheim (DE)**

(72) Erfinder: **AYGEN, Sitke**
**51105 Köln (DE)**

(74) Vertreter: **Schrell, Andreas**
**Gleiss Große Schrell und Partner mbB**
**Patentanwälte Rechtsanwälte**
**Leitzstrasse 45**
**70469 Stuttgart (DE)**

(56) Entgegenhaltungen:
**EP-B1- 1 415 159    WO-A2-2006/028648**
**JP-A- 55 069 038    US-A- 5 542 419**
**US-A1- 2008 090 268**

- **TUCHMAN MENDEL ET AL: "N-carbamylglutamate markedly enhances ureagenesis in N-acetylglutamate deficiency and propionic acidemia as measured by isotopic incorporation and blood biomarkers", PEDIATRIC RESEARCH, WILLIAMS AND WILKINS, BALTIMORE, MD, US, Bd. 64, Nr. 2, 1. August 2008 (2008-08-01) , Seiten 213-217, XP008127178, ISSN: 0031-3998, DOI: DOI: 10.1203/PDR.0B013E318179454B in der Anmeldung erwähnt**
- **CHEY W D ET AL: "The 13C-urea blood test accurately detects active Helicobacter pylori infection: a US, Multicenter trial", AMERICAN JOURNAL OF GASTROENTEROLOGY, ELSEVIER SCIENCE INC, US, Bd. 94, Nr. 6, 1. Januar 1999 (1999-01-01) , Seiten 1522-1524, XP002245806, ISSN: 0002-9270, DOI: DOI: 10.1111/J.1572-0241.1999.1137_R.X**

EP 2 606 147 B1

**Beschreibung**

Die vorliegende Erfindung betrifft ein Verfahren zur Isolierung von Harnstoff in Blutproben.

Harnstoff ist eine organische Verbindung, die im menschlichen Organismus ein Endprodukt des Stoffwechsels von Stickstoffverbindungen darstellt. Beim Menschen wird Harnstoff mit dem Urin ausgeschieden.

Die Harnstoffbildung erfolgt überwiegend in den Leberzellen und teilweise in den Nieren. Bei der Harnstoffbildung im Körper gibt es verschiedene Erkrankungen, teilweise erblich, die erhebliche gesundheitliche Schäden bewirken können. Die Bestimmung der Harnstoffbildung ist ein Indikator für die Funktion der Leber, beispielsweise bei Lebertransplantationen oder der Transplantation von Leberzellen.

Tuchman et al., Pediatric Research 2008 (64), Seite 213 beschreiben beispielsweise einen Defekt der N-Acetylglutamat-Synthase und die Analyse der Harnstoffbildung.

Zur Messung der Harnstoffbildung erhielten Patienten oral $^{13}$C markiertes Natriumacetat verabreicht, das im Körper zur Bildung von $^{13}$C markiertem Harnstoff führte; aus $^{13}$C markiertem Acetat entsteht $^{13}CO_2$, das in $^{13}$C-Carbamoyl-Phosphat und anschließend in $^{13}$C Harnstoff überführt wird.

Die meisten chemischen Elemente existieren in der Natur als Gemische mehrerer stabiler oder radioaktiver Isotope. Isotopenhäufigkeiten werden normalerweise auch bei Tracerstudien mit angereicherten Verbindungen in der Einheit Atomprozent (atom-%) oder ppm angegeben. Zur Beschreibung der Variationen im Bereich natürlicher Häufigkeiten existiert die relative Delta-Skala in Promille (‰). Die $\delta$-Werte (z.B. $\delta^{13}$C, $\delta^{15}$N, $\delta^{18}$O) werden als Differenz des jeweiligen Isotopenverhältnisses R ([schweres Isotop]/[leichtes Isotop], z.B. $R^{13}C=[^{13}C]/[^{12}C]$ ) der Probe gegenüber einem Standard, relativ zu diesem Standard definiert.

Der $\delta^{13}$C-Wert berechnet sich z.B. nach:

$$\delta^{13}C \;=\; \frac{R_{Probe}-R_{Standard}}{R_{Standard}} \cdot 1000 \;=\; \left(\frac{R_{Probe}}{R_{Standard}}-1\right)\cdot 1000$$

Der Standard für Kohlenstoff ist ein Kalk, PDB (Pee Dee Belemnite). Der durch den Einbau von $CO_2$ in der Photosynthese gebundene Kohlenstoff ist generell an $^{13}$C abgereichert. Die meisten Pflanzen reduzieren $CO_2$ zu Kohlenhydraten nach dem Calvin-Benson- oder C3-Weg. Dies führt dazu, dass die Biomasse von C3-Pflanzen (zu diesen gehören die Nutzpflanzen Reis, Kartoffel, Soja, Zuckerrübe und Getreide) $\delta^{13}$C-Werte im Bereich -24 bis -32‰o aufweisen. Andere Pflanzen fixieren $CO_2$ nach dem Hatch-Slack- oder C4-Weg. Die $\delta^{13}$C-Werte von Produkten aus C4-Pflanzen (Mais, Hirse, Zuckerrohr) haben $\delta^{13}$C-Werte im Bereich -10 bis -16‰. Deswegen können $\delta^{13}$C-Werte zur Überprüfung der Herkunft und Originalität von organischen Substanzen verwendet werden.

Die Bestimmung des $\delta^{13}$C-Wertes von Plasma-Harnstoff erfolgt üblicherweise durch eine Umsetzung des Harnstoffs in $CO_2$ mit Hilfe eines Enzyms. Deshalb ist es wichtig, dass die aus Plasma isolierte Harnstoff-Lösung frei von fremdem $CO_2$ ist. Allerdings ist in der Regel immer $CO_2$ im Plasma vorhanden, entweder als gelöstes freies $CO_2$ oder als gebundenes $CO_2$ in der Form von Bikarbonat. Eine vollständige Befreiung des Plasma von $CO_2$ ist nicht einfach; zusätzlich muss verhindert werden, bei der Isolierung $CO_2$ in die Probe einzutragen.

Zur Isolierung des Harnstoffes aus Blutplasma verwenden Tuchman et al. ein Verfahren mit folgenden Schritten:

Eine Plasmaprobe von 0,5 ml wurde mit 0,5 ml $H_2O$ und 40 $\mu l$ Perchlorsäure 60% versetzt und präzipitiertes Protein abgetrennt. Anschließend ruhte der Behälter 30 Minuten, um eine Freisetzung von $CO_2$ zu erlauben. Nach Überführen in ein neues Gefäß und Einstellen des pH in den Bereich 6 bis 7 mit 300 $\mu l$ KOH 1 M wurde präzipitiertes Kaliumperchlorat abgetrennt. Mit Hilfe einer Ionenaustauschersäule wurde restliches Bicarbonat entfernt.

Die Säule wurde mit 1 ml HCl 10 mM gewaschen und das Eluat in einem Glasbehälter bei 80°C getrocknet. Die Probe ruhte über Nacht in einem verschlossenen Behälter, in dem ein Stück Gaze, das mit Natriumhydroxid getränkt war, mit eingeschlossen war, um Reste von $CO_2$ abzufangen.

Anschließend wurde der Behälter mit Helium gespült und das Gefäß mit einem Gummistopfen luftdicht verschlossen. Es wurden 400 $\mu l$ Kaliumphosphatpuffer 0.5 M pH 6.0 mit 3 mg Urease-Enzym/400 $\mu l$ durch den Gummistopfen injiziert. Nach einer Stunde erfolgte eine Zugabe von 100 $\mu L$ Phosphorsäure 20%, um $CO_2$ freizusetzten und die Urease-Reaktion zu stoppen. Das freigesetzte $^{13}CO_2$ wurde mit Hilfe eines isotope ratio mass spectrometer (IRMS) vermessen.

Die Untersuchungen der Anmelderin haben ergeben, dass das oben beschriebene Verfahren sehr empfindlich ist. Mehrere Quellen von Fremd-$CO_2$ können bei diesem Verfahren die gemessenen delta-Werte des aus Harnstoff entstandenen $CO_2$ verfälschen. Es ist darüber hinaus auch sehr aufwändig und langwierig. Wegen der niedrigen Ausbeute an Harnstoff ist ein größeres Volumen (0,5 ml) von Plasma notwendig. Dies kann bei Kindern Probleme hervorrufen. Auch bei einer Crossvalidierung mit USA und Europa haben sich nicht nachvollziehbare Differenzen in den Ergebnissen gezeigt.

Dass bei dem Tuchman et al. beschriebenen Verfahren zur Harnstoffisolierung aus Plasma die Entfernung von störendem $CO_2$ nicht vollständig gelingt, kann man durch den Vergleich der $\delta^{13}C$-Werte von aus Harnstoff erzeugtes $CO_2$ nachweisen. Die von Tuchmann et al. beobachteten $\delta^{13}C$-Werte sind sehr niedrig -25 bis -26‰.

Der natürliche Harnstoff in Plasma hat einen $\delta^{13}C$-Wert von -19 bis -23‰, abhängig von der Ernährung.

Aufgabe der Erfindung war es, ein Verfahren bereitzustellen, das zumindest einige der Nachteile des bekannten Verfahrens überwindet.

Gelöst wird die Aufgabe durch ein Verfahren zur Isolierung von Harnstoff und Entfernung von $CO_2$ in Plasmaproben, umfassend folgende Schritte:

a) Bereitstellen einer Plasmaprobe enthaltend Harnstoff

b) Zugabe einer Säure, um $CO_2$ teilweise zu entfernen

c) Gefriertrocknen der Probe, um $CO_2$ zu entfernen und eine getrocknete Probe zu erhalten

d) Wiederauflösen der getrockneten Probe und Neutralisieren auf einen pH-Wert von 4 bis 7, vorzugsweise 4 bis 6,9, insbesondere 4 bis 6, mit einer Pufferlösung

Ausgangspunkt für die Isolierung des Harnstoffs in einer Plasmaprobe ist eine Plasmaprobe, die Harnstoff enthält. Plasmaproben können in bekannter Weise aus Blutproben gewonnen werden. Auf Grund der hohen Reproduzierbarkeit des erfindungsgemäßen Verfahrens genügen Plasmaproben mit einem Volumen im Bereich von 0,2 bis 0,3 ml, es können aber auch größere Mengen eingesetzt werden.

Die Plasmaprobe enthält zumindest Harnstoff mit einem natürlichen Isotopenverhältnis. Sie kann zusätzlich mit $^{13}C$ angereichertem Harnstoff versetzt sein. Sie kann aber auch durch Verabreichung von $^{13}C$ markierten Vorstufen, beispielsweise Acetat oder Bicarbonat, mit $^{13}C$ angereichert sein. Hiervon können verträgliche Salze, z.B. Na oder K-Salze verabreicht werden.

Erfindungsgemäß wird der Harnstoff gemessen durch Umsetzung des Harnstoffs mit Urease zur Freisetzung von $CO_2$, daher muss zunächst vorhandenes $CO_2$ entfernt werden.

In einer Ausführungsform der Erfindung wird zunächst eine Filtration durchgeführt. Hierzu wird zur Fällung Lösungsmittel, beispielsweise Acetonitril und/oder Ameisensäure zugesetzt. Durch diese Filtration können Proteine und Lipide vom Plasma abgetrennt werden. Besonders geeignet hierfür sind sogenannte HybridSPE™ Säulen, die von der Firma SUPELCO erhältlich sind. Diese eignen sich insbesondere auch zu Entfernung von Phospholipiden.

Es hat sich jedoch gezeigt, dass auf den Schritt der Abtrennung der Proteine und Lipide aus dem Plasma auch verzichtet werden kann und trotzdem sehr gut reproduzierbare Werte erhalten werden. Der Verzicht auf den Filtrationsschritt spart zum einen Zeit, zum anderen aber auch Kosten, die für entsprechende Filter anfallen.

Erfindungsgemäß wird eine Säure zugesetzt. Durch den Zusatz an Säure wird ein Teil des $CO_2$ aus der Plasmaprobe entfernt. Geeignet ist beispielsweise Phosphorsäure in einer Konzentration von etwa 20%. Bezogen auf eine Plasmaprobe von 0,3 ml genügt die Säure (z.B. Phosphorsäure) in einer Menge von etwa 50 µl. Selbstverständlich können auch andere Säuren eingesetzt werden.

Ein wesentlicher Schritt des erfindungsgemäßen Verfahrens ist die anschließende Gefriertrocknung der Probe. Gefriertrocknen ist ein Verfahren, bei dem eine Probe eingefroren wird und im Vakuum das enthaltende Wasser sublimiert. Erfindungsgemäß eignet sich dieses Verfahren hervorragend zur Entfernung von Restmengen $CO_2$ in der Probe.

Die erhaltene Probe wird anschließend wieder aufgelöst und auf einen pH-Wert im Bereich von 4 bis 7, vorzugsweise 4 bis 6,9, insbesondere 4 bis 6, vorzugsweise 5 bis 6 eingestellt. Zur Einstellung eignen sich insbesondere Pufferlösungen, beispielsweise Phosphatpufferlösungen mit einem pH von 9,0. Auch andere Pufferlösungen können eingesetzt werden. Die in Tuchman et al. eingesetzte Kalilauge ist besonders ungünstig, da sie teilweise größere Mengen $CO_2$ enthält.

In einer Ausführungsform der Erfindung wird nach dem Wiederauflösen der getrockneten Proben wird diese entgast, um Reste von Fremd-$CO_2$, insbesondere aus der Zugabe des Puffers auszutreiben. Geeignet hat sich das Anlegen eines Vakuums im Bereich von 1 bis 10 mbar für 2 bis 3 Stunden.

Das Ergebnis dies Verfahrens ist eine Probe, die den Harnstoff aus dem Plasma noch enthält, aber im wesentlichen frei ist von $CO_2$ aus dem Plasma.

Zur Bestimmung des Isotopenverhältnisses des Harnstoffs wird nun auf ein an sich bekanntes Verfahren die Umwandlung mit Urease, zurückgegriffen:

Die wieder aufgelöste Probe wird mit einem Schutzgas gespült, beispielsweise Helium, Stickstoff oder Argon und gasdicht verschlossen. Anschließend erfolgt eine Zugabe von Urease, um aus dem vorhandenen Harnstoff $CO_2$ zu bilden. Geeignet ist beispielsweise eine Urease, wie sie von der Firma Sigma erhältlich ist. Eine Menge von 20 bis 100 Einheiten Urease für eine Plasmaprobe von 0,3 ml hat sich als besonders geeignet erwiesen.

Anschließend erfolgt eine Inkubation der Lösung. Die Inkubation bei einer Temperatur von etwa 36°C für einen Zeitraum von etwa 60 Minuten hat sich als geeignet erwiesen.

Danach wird eine Säure zugesetzt, um die weitere Ureasereaktion zu stoppen. Zusätzlich wird durch die Zugabe der Säure das gebildete $CO_2$ freigesetzt. Im Hinblick auf die 0,3 ml große Plasmaprobe hat sich die Verwendung von 100 $\mu$l Phosphorsäure 20%ig als geeignet erwiesen. In dem gasdichten Behälter ist nun $CO_2$ freigesetzt worden, das aus der Umsetzung des Harnstoffs im Plasma stammt. Nun kann das Isotopenverhältnis des $CO_2$ bestimmt werden. Für diese Bestimmung eignet sich insbesondere IRMS. Bei der IRMS wird das Verhältnis zwischen $^{13}C$ und $^{12}C$ relativ zu einem Standard gemessen, wie oben ausgeführt.

Zur Prüfung der Durchführung kann es sinnvoll sein, vor der Zugabe von Urease zu prüfen, ob die Lösung frei von $CO_2$ ist. Dies kann beispielsweise mittels IR-MS Spektroskopie erfolgen.

In einer Anwendung der Erfindung werden zur Messung der Kinetik der Harnstoffbildung $^{13}C$ markierte Substanzen dem Patienten verabreicht, um die Bildung von $^{13}C$ markiertem Harnstoff zu bestimmen. In einer bevorzugten Ausführungsform der Erfindung wird eine Kinetik dadurch bestimmt, dass zunächst eine Blutprobe entnommen wird, bevor ein Patient $^{13}C$ markierte Harnstoffvorläufer einnimmt (Basalwert), gefolgt einer oder bevorzugt mehrere Blutprobennahme, nachdem der Patient $^{13}C$-markierte Harnstoffvorläufer eingenommen hat. Hierdurch kann die Bildung von $^{13}C$ markiertem Harnstoff überprüft werden.

Erfindungsgemäß können Plasmamengen von etwa 100 bis 200 oder 200 bis 300 ml eingesetzt werden, d.h. im Vergleich zum Verfahren von Tuchman, genügt eine Blutprobe mit halber Größe. Insbesondere wenn eine Kinetik bestimmt wird und das Verfahren bei Kindern angewandt wird, ist es vorteilhaft, wenn die Mengen an genommenem Blut besonders klein sind. Gegenüber dem Verfahren von Tuchman et al. hat das erfindungsgemäße Verfahren eine bessere Reproduzierbarkeit und ist weniger aufwändig.

Die vorliegende Erfindung betrifft in besonders bevorzugter Ausführungsform die folgenden Aspekte:

Aspekt 1: Verfahren zur Isolierung von Harnstoff und Entfernung von $CO_2$ aus Plasmaproben umfassend folgende Schritte a) Bereitstellen einer Plasmaprobe, b) Zugabe einer Säure, um $CO_2$ teilweise zu entfernen, c) Gefriertrocknen der Probe, um weiteres $CO_2$ zu entfernen und eine getrocknete Probe zu erhalten und d) Wiederauflösen der getrockneten Probe und Neutralisieren auf einen pH-Wert von 4 bis 7, vorzugsweise 4 bis 6,9, insbesondere 4 bis 6, mit einer Pufferlösung.

Aspekt 2: Verfahren nach Aspekt 1, dadurch gekennzeichnet, dass vor der Zugabe einer Säure in Schritt b) eine Filtration durchgeführt wird.

Aspekt 3: Verfahren nach Aspekt 1 oder 2, dadurch gekennzeichnet, dass nach Schritt d) die Probe unter reduziertem Druck entgast wird.

Aspekt 4: Verfahren nach mindestens einem der Aspekte 1 bis 3, dadurch gekennzeichnet, dass die Probe in Schritt d) auf einen pH-Wert von 5 bis 6 eingestellt wird.

Aspekt 5: Verfahren nach mindestens einem der Aspekte 1 bis 4, dadurch gekennzeichnet, dass die Plasmaprobe von einem Probanden oder Patienten stammt, der vor der Entnahme $^{13}C$-markierte Harnstoffvorläufer eingenommen hat.

Aspekt 6: Verfahren zur Bestimmung des $^{13}C$-Isotopenverhältnisses von Harnstoff in einer Plasmaprobe umfassend die Schritte Isolierung von Harnstoff durch ein Verfahren nach einem der Aspekte 1 bis 5, Spülen mit Schutzgas, Zugabe von Urease, um $CO_2$ zu bilden, Inkubation, Zugabe einer Säure, um das gebildete $CO_2$ freizusetzen und Messung des $^{13}C$-Isotopenverhältnisses des freigesetzten $CO_2$.

Aspekt 7: Verfahren zur Diagnostik des Harnstoffstoffwechsels umfassend die Schritte Bereitstellen einer ersten Plasmaprobe eines Patienten, Bestimmung des $^{13}C$-Isotopenverhältnisses von Harnstoff in der ersten Plasmaprobe gemäß Aspekt 6, Bereitstellen von mindestens einer weiteren Plasmaprobe, die von dem Patienten stammt, wobei der Patient vor der Entnahme $^{13}C$-markierte Harnstoffvorläufer eingenommen hat, Bestimmung des $^{13}C$-Isotopenverhältnisses von Harnstoff in der mindestens einen weiteren Plasmaprobe gemäß Aspekt 6 und Quantifizierung der Menge des gebildeten Harnstoffs durch das $^{13}C$-Isotopenverhältnis des Harnstoffs in der ersten und der mindestens einen weiteren Plasmaprobe.

Aspekt 8: Verfahren nach Aspekt 7, dadurch gekennzeichnet, dass mindestens zwei weitere Plasmaproben verwendet werden.

Aspekt 9: Verfahren nach Aspekt 8, dadurch gekennzeichnet, dass die weiteren Plasmaproben im Zeitraum von 15 bis 240 min entnommen wurden, nachdem der Patient $^{13}$C-markierte Harnstoffvorläufer eingenommen hatte.

Aspekt 10: Verfahren nach einem der Aspekte 8 oder 9, dadurch gekennzeichnet, dass die weiteren Plasmaproben im Abstand von 15 min entnommen wurden, nachdem der Patient $^{13}$C-markierte Harnstoffvorläufer eingenommen hatte.

Die vorliegende Erfindung betrifft insbesondere auch die folgenden Aspekte A), B), C), D), E), F), G), H), I):

Aspekt A): Verfahren zur Bestimmung von Harnstoff in Plasmaproben umfassend folgende Schritte a) Bereitstellen einer Plasmaprobe enthaltend Harnstoff, b) Zugabe einer Säure, c) Gefriertrocknen der Probe, um $CO_2$ zu entfernen und eine getrocknete Probe zu erhalten, d) Wiederauflösen der getrockneten Probe und Neutralisieren auf einen pH-Wert von 5 bis 7 mit einer Pufferlösung, e) Spülen mit Schutzgas, f) Zugabe von Urease, um $CO_2$ zu bilden, g) Inkubation, h) Zugabe einer Säure, um das gebildete $CO_2$ freizusetzen und i) Bestimmung des Isotopenverhältnisses des freigesetzten $CO_2$.

Aspekt B): Verfahren nach Aspekt A), dadurch gekennzeichnet, dass vor dem Gefriertrocknen eine Filtration durchgeführt wird.

Aspekt C): Verfahren nach Aspekt A) oder B), dadurch gekennzeichnet, dass nach Schritt d) die Probe eine Temperatur zwischen 40 und 70°C erwärmt wird.

Aspekt D): Verfahren nach mindestens einem der Aspekte A) bis C), dadurch gekennzeichnet, dass zur Bestimmung des Isotopenverhältnisses des CO2 IRMS eingesetzt wird.

Aspekt E): Verfahren nach mindestens einem der Aspekte A) bis D), dadurch gekennzeichnet, dass vor der Entnahme einer Plasmaprobe 13C -markiertes Acetat verabreicht wird.

Aspekt F): Verfahren nach mindestens einem der Aspekte A) bis E), dadurch gekennzeichnet, dass die Probe in Schritt d) auf einen pH-Wert von 5 bis 6 eingestellt wird.

Aspekt G): Verfahren zur Diagnostik des Harnstoffstoffwechsels umfassend die Schritte Entnehmen einer Blutprobe von einem Patienten, Gewinnung einer Plasmaprobe aus der Blutprobe, Bestimmung des Harnstoffs in der Plasmaprobe nach dem Verfahren gemäß einem der Aspekte A) bis F), Verabreichen eines 13C markierten Harnstoffvorläufers, Entnahme von mindestens zwei Blutproben in einem zeitlichen Abstand und Bestimmung des Harnstoffs nach Gewinnung von Plasmaproben aus den Blutproben durch ein Verfahren nach einem der Aspekte A) bis F).

Aspekt H): Verfahren nach Aspekt G), dadurch gekennzeichnet, dass nach dem Verabreichen der Harnstoffvorläufer, die mindestens zwei Blutproben über einen Zeitraum von mindestens 120 Minuten, bevorzugt mindestens 240 Minuten entnommen werden.

Aspekt I): Verfahren nach Aspekt G) oder H), dadurch gekennzeichnet, dass zwischen zwei Entnahmen von Blutproben ein Zeitraum von 10 bis 20 Minuten liegt.

*Figurenbeschreibung*

Figur 1 zeigt die Reproduzierbarkeit des Verfahrens gemäß Beispiel 3.

Figur 2 zeigt die Eichkurve des Spiking-Experiments gemäß Beispiel 4.

Figur 3 zeigt die Reproduzierbarkeit des Verfahrens gemäß Beispiel 5.

Figur 4 zeigt die Eichkurve des Spiking-Experiment gemäß Beispiel 6.

Figuren 5 und 6 zeigen die Ergebnisse der Messung der Harnstoffbildung gemäß Beispiel 7.

Das Verfahren wird durch die folgenden Beispiele näher erläutert.

### Beispiel 1: Harnstoffisolation mit Filtration

Aus einer Blutprobe wurde Plasma gewonnen. 300 $\mu$l Plasma wurden mit 200 $\mu$l deionisiertem Wasser verdünnt. Zu der Probe wurden 100 $\mu$l Acetonitril mit 1% Ameisensäure zugesetzt. In dem Gefäß bildete sich ein Niederschlag. Die Proben wurden über eine HybridSPE-Säule abfiltriert.

Das Filtrat wurde mit 50 $\mu$l Phosphorsäure 1 M versetzt, eingefroren und lyophilisiert. Die Probe wurde mit einer entgasten Phosphatpufferlösung 0,5 M pH 9 auf einen pH-Wert von 5,5 eingestellt. Der Probenbehälter (Vacutainer) wurde mit Heliumgas gespült. Anschließend erfolgte die Zugabe von 70 $\mu$l einer Lösung enthaltend 15 mg/ml Jack Bean Urease Typ III der Firma Sigma in Phosphatpuffer. Die Probe wurde für eine Stunde bei 36°C inkubiert. Anschließend wurden durch das Septum 60 $\mu$l Phosphorsäure 20%ig injiziert, um die Ureasereaktion zu stoppen und $CO_2$ freizusetzen. Aus dem freigesetzten $CO_2$ erfolgte die Bestimmung des Isotopenverhältnisses mittels IRMS.

### Beispiel 2: Harnstoffisolation ohne **Filtration**

Das Verfahren wurde wie in Beispiel 1 durchgeführt, allerdings wurde auf die Zugabe von Acetonitril und Ameisensäure sowie die Filtration verzichtet, d.h. die Plasmaprobe wurde nach Zugabe von Säure direkt lyophilisiert. Das weitere Verfahren wurde identisch durchgeführt.

### Beispiel 3: Reproduzierbarkeit des Verfahrens

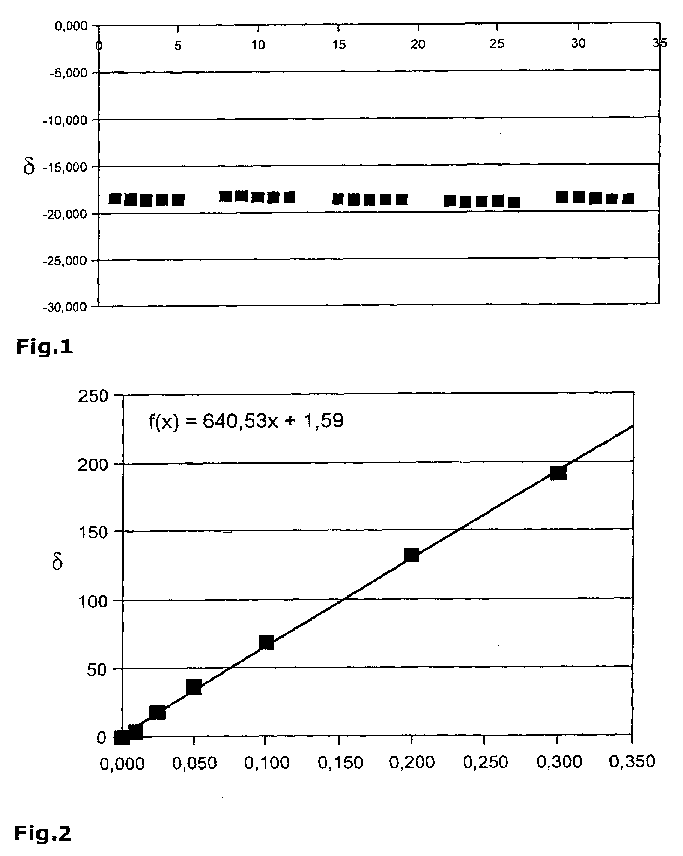

Eine Plasmaprobe wurde in fünf Proben unterteilt, die jeweils getrennt voneinander dem Verfahren gemäß Beispiel 1 unterzogen wurden. Jede erhaltene $CO_2$ Probe wurde fünfmal gemessen. Die Unterschiede sind minimal, vgl. Figur 1.

### Beispiel 4: Spiking-Experiment

Den Plasmaproben aus Beispiel 1 wurden 99 %ig markierter $^{13}$C Harnstoff in Mengen von 0,01 mg, 0,25 mg, 0,5 mg, 0,1 mg, 0,2 mg, 0,3 mg zugesetzt und die Probe gemäß Verfahren 1 behandelt. Figur 2 zeigt die entsprechenden Messwerte. Die Eichkurve liegt auf einer Geraden mit einem Korrelationskoeffizienten von 0,99923.

### Beispiel 5: Reproduzierbarkeit

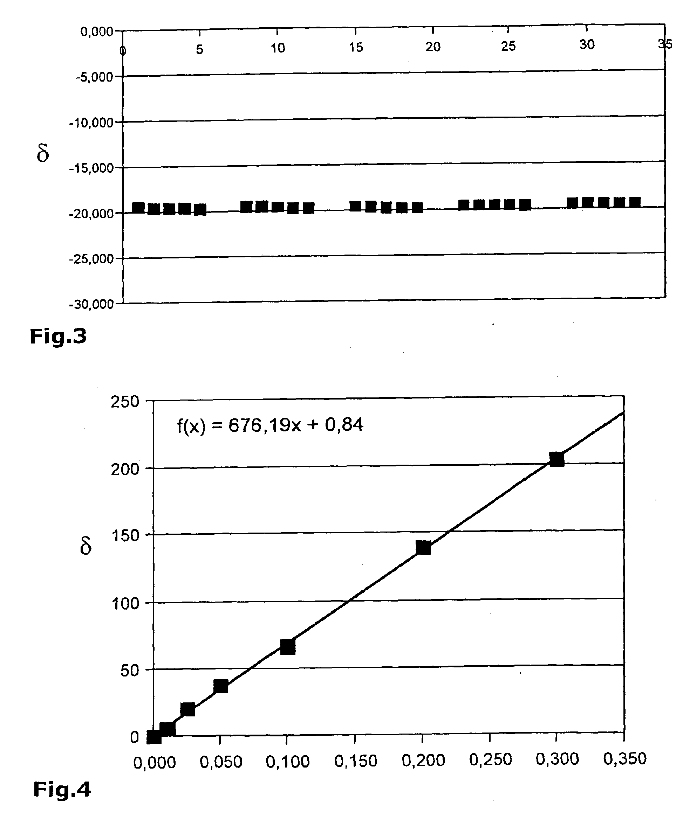

Die Messung der Reproduzierbarkeit gemäß Beispiel 3 wurde für das Verfahren ohne Filter gemäß Beispiel 2 wiederholt. Figur 3 zeigt die Ergebnisse. Auch hier besteht eine hervorragende Reproduzierbarkeit.

### Beispiel 6: Spiking-Experiment

Das Spiking-Experiment gemäß Beispiel 4 wurde wiederholt, wobei das Verfahren gemäß Beispiel 2 eingesetzt wurde. Die entsprechende Eichgerade ist in Figur 4 ersichtlich. Ihr Korrelationskoeffizient lag bei R=0,99959.

### Beispiel 7: Messung der Harnstoffbildung

Zwei Probanden wurden Blut entnommen und Plasma hergestellt aus je 300 $\mu$l Plasma wurde gemäß dem erfindungsgemäßen Verfahren Harnstoff isoliert und das $^{13}$C/$^{12}$C- Isotopenverhältnis des Harnstoffs bestimmt.

Dabei wurde jede Probe 5-mal gemessen und über den Durchschnittwert der Basalwert ermittelt. Danach wurde den Probanden 27 mg/kg 99% $^{13}$C markiertes Na-Acetat verabreicht.

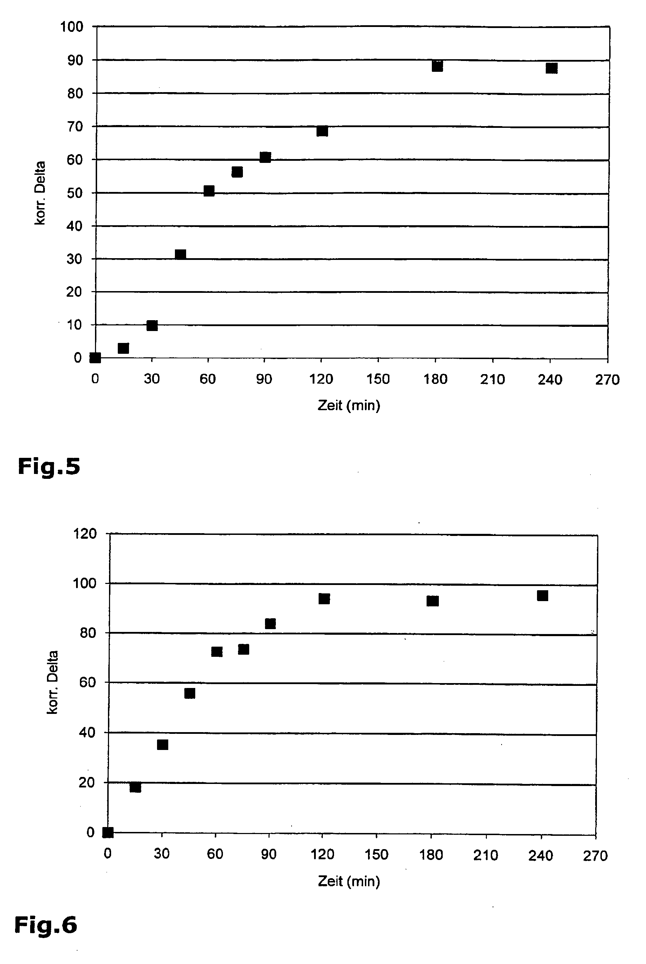

Die Prozedur der Blutentnahme und die Bestimmung des $^{13}$C/$^{12}$C- Isotopenverhältnisses des isolierten Harnstoffs wurde in 15, 30, 45, 60, 75, 90, 120, 180 und 240 Minuten Abstand wiederholt. Ein kleinerer Anteil des $^{13}$C markiertes Acetats wird im Körper in Harnstoff umgewandelt und ist aufgrund der Empfindlichkeit der Messung nachweisbar. Die Kinetik des neu gebildeten Harnstoffs wurde durch Messung der Delta Werte (Erhöhung der $^{13}$C/$^{12}$C- Isotopenverhältnisses) bestimmt. Die Kinetik der Harnstoffbildung ist in den Figuren 5 und 6 dargestellt.

# EP 2 606 147 B1

**Patentansprüche**

1. Verfahren zur Isolierung von Harnstoff und Entfernung von $CO_2$ aus Plasmaproben umfassend folgende Schritte

   a) Bereitstellen einer Plasmaprobe
   b) Zugabe einer Säure, um $CO_2$ teilweise zu entfernen
   c) Gefriertrocknen der Probe, um weiteres $CO_2$ zu entfernen und eine getrocknete Probe zu erhalten
   d) Wiederauflösen der getrockneten Probe und Neutralisieren auf einen pH-Wert von 4 bis 7 mit einer Pufferlösung.

2. Verfahren nach Anspruch 1, **dadurch gekennzeichnet, dass** vor der Zugabe einer Säure in Schritt b) eine Filtration durchgeführt wird.

3. Verfahren nach Anspruch 1 oder 2, **dadurch gekennzeichnet, dass** nach Schritt d) die Probe unter reduziertem Druck entgast wird.

4. Verfahren nach mindestens einem der Ansprüche 1 bis 3, **dadurch gekennzeichnet, dass** die Probe in Schritt d) auf einen pH-Wert von 4 bis 6, vorzugsweise 5 bis 6, eingestellt wird.

5. Verfahren nach mindestens einem der Ansprüche 1 bis 4, **dadurch gekennzeichnet, dass** die Plasmaprobe von einem Probanden oder Patienten stammt, der vor der Entnahme $^{13}$C-markierte Harnstoffvorläufer eingenommen hat.

6. Verfahren zur Bestimmung des $^{13}$C-Isotopenverhältnisses von Harnstoff in einer Plasmaprobe umfassend die Schritte

   - Isolierung von Harnstoff durch ein Verfahren nach einem der Ansprüche 1 bis 5
   - Spülen mit Schutzgas
   - Zugabe von Urease, um $CO_2$ zu bilden
   - Inkubation
   - Zugabe einer Säure, um das gebildete $CO_2$ freizusetzen
   - Messung des $^{13}$C-Isotopenverhältnisses des freigesetzten $CO_2$.

7. Verfahren zur Diagnostik des Harnstoffstoffwechsels umfassend die Schritte

   - Bereitstellen einer ersten Plasmaprobe eines Patienten,
   - Bestimmung des $^{13}$C-Isotopenverhältnisses von Harnstoff in der ersten Plasmaprobe gemäß Anspruch 6,
   - Bereitstellen von mindestens einer weiteren Plasmaprobe, die von dem Patienten stammt, wobei der Patient vor der Entnahme $^{13}$C-markierte Harnstoffvorläufer eingenommen hat,
   - Bestimmung des $^{13}$C-Isotopenverhältnisses von Harnstoff in der mindestens einen weiteren Plasmaprobe gemäß Anspruch 6,
   - Quantifizierung der Menge des gebildeten Harnstoffs durch das $^{13}$C-Isotopenverhältnis des Harnstoffs in der ersten und der mindestens einen weiteren Plasmaprobe.

8. Verfahren nach Anspruch 7, **dadurch gekennzeichnet, dass** mindestens zwei weitere Plasmaproben verwendet werden.

9. Verfahren nach Anspruch 8, dadurch gekennzechnet, dass die weiteren Plasmaproben im Zeitraum von 15 bis 240 min erhalten, nachdem der Patient $^{13}$C-markierte Harnstoffvorläufer eingenommen hatte.

10. Verfahren nach einem der Ansprüche 8 oder 9, **dadurch gekennzeichnet, dass** die weiteren Plasmaproben im Abstand von 15 min erhalten wurden, nachdem der Patient $^{13}$C-markierte Harnstoffvorläufer eingenommen hatte.

**Claims**

1. A method for isolating urea and removing $CO_2$ from plasma samples, comprising the following steps:

   a) providing a plasma sample

b) adding an acid so as to partially remove $CO_2$

c) lyophilizing the sample so as to further remove $CO_2$ and obtain a dried sample

d) redissolving the dried sample and neutralizing to a pH value of 4 to 7 using a buffer solution.

2. The method according to claim 1, **characterized in that** a filtration step is carried out before adding an acid in step b).

3. The method according to claim 1 or 2, **characterized in that**, after step d), the sample is degassed at reduced pressure.

4. A method according to at least one of claims 1 to 3, **characterized in that**, in step d), the sample is adjusted to a pH value of 4 to 6, preferably 5 to 6.

5. A method according to at least one of claims 1 to 4, **characterized in that** the plasma sample originates from a subject or patient who took $^{13}$C-labeled urea precursors before collection.

6. A method for determining the $^{13}$C isotope ratio of urea in a plasma sample, comprising the following steps:

- isolating urea by way of a method according to any one of claims 1 to 5
- rinsing with inert gas
- adding urease so as to produce $CO_2$
- incubating
- adding an acid so as to release the $CO_2$ that has been produced
- measuring the $^{13}$C isotope ratio of the released $CO_2$.

7. A method for diagnosing urea metabolism, comprising the following steps:

- providing a first plasma sample of a patient;
- determining the $^{13}$C isotope ratio of urea in a first plasma sample according to claim 6;
- providing at least one additional plasma sample originating from the patient, wherein the patent took $^{13}$C-labeled urea precursors before collection;
- determining the $^{13}$C isotope ratio of urea in the at least one additional plasma sample according to claim 6;
- quantifying the amount of urea that has been produced by way of the $^{13}$C isotope ratio of the urea in the first and the at least one additional plasma samples.

8. The method according to claim 7, **characterized in that** at least two additional plasma samples are used.

9. The method according to claim 8, **characterized in that** the additional plasma samples were obtained over a period of 15 to 240 minutes after the patient took $^{13}$C-labeled urea precursors.

10. The method according to claim 8 or 9, **characterized in that** the additional plasma samples were obtained at intervals of 15 minutes after the patient took $^{13}$C-labeled urea precursors.


**Revendications**

1. Procédé pour isoler de l'urée et pour éliminer de $CO_2$ des échantillons de plasma, le procédé comprenant les étapes suivantes :

a) fourniture d'un échantillon de plasma

b) addition d'un acide, afin de partiellement éliminer de $CO_2$

c) lyophilisation de l'échantillon, afin d'éliminer d'autre $CO_2$ et d'obtenir un échantillon séché

d) redissolution de l'échantillon séché et neutralisation jusqu'à une valeur pH de 4 à 7 avec une solution tampon.

2. Procédé selon la revendication 1, **caractérisé en ce qu'**une filtration est effectuée avant l'addition d'un acide dans l'étape b).

3. Procédé selon la revendication 1 ou 2, **caractérisé en ce que** l'échantillon est dégazé sous pression réduite après l'étape d).

4. Procédé selon au moins une des revendications 1 à 3, **caractérisé en ce que** dans l'étape d) l'échantillon est ajusté à une valeur pH de 4 à 6, de préférence de 5 à 6.

5. Procédé selon au moins une des revendications 1 à 4, **caractérisé en ce que** l'échantillon de plasma est obtenu d'un sujet ou patient qui a pris des précurseurs d'urée marqués au carbone 13 avant l'échantillonnage.

6. Procédé pour la détermination du rapport isotopique du carbone 13 de l'urée dans un échantillon de plasma, le procédé comprenant les étapes suivantes :

   - isolation de l'urée par un procédé selon l'une quelconque des revendications 1 à 5
   - rinçage avec gaz protecteur
   - addition de l'uréase afin de former de $CO_2$
   - incubation
   - addition d'un acide afin de libérer le $CO_2$ formé
   - mesure du rapport isotopique du carbone 13 du $CO_2$ libéré.

7. Procédé pour le diagnostic du métabolisme de l'urée, le procédé comprenant les étapes suivantes :

   - fourniture d'un premier échantillon de plasma d'un patient,
   - détermination du rapport isotopique du carbone 13 de l'urée dans le premier échantillon de plasma selon la revendication 6,
   - fourniture d'au moins un autre échantillon de plasma obtenu du patient, dans lequel le patient a pris des précurseurs d'urée marqués au carbone 13 avant l'échantillonnage,
   - détermination du rapport isotopique du carbone 13 de l'urée dans l'au moins un autre échantillon de plasma selon la revendication 6,
   - quantification de la quantité de l'urée formée à partir du rapport isotopique du carbone 13 de l'urée dans le premier et l'au moins un autre échantillon de plasma.

8. Procédé selon la revendication 7, **caractérisé en ce qu'**au moins deux autres échantillons de plasma sont utilisés.

9. Procédé selon la revendication 8, **caractérisé en ce que** les autres échantillons de plasma sont obtenus dans la période de 15 à 240 min après que le patient a pris des précurseurs d'urée marqués au carbone 13.

10. Procédé selon l'une quelconque des revendications 8 ou 9, **caractérisé en ce que** les autres échantillons de plasma sont obtenus à des intervalles de 15 min après que le patient a pris des précurseurs d'urée marqués au carbone 13.

**Fig.1**

f(x) = 640,53x + 1,59

**Fig.2**

**Fig.3**

f(x) = 676,19x + 0,84

**Fig.4**

**Fig.5**

**Fig.6**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **TUCHMAN et al.** *Pediatric Research,* 2008, 213